# EUROPEAN PATENT SPECIFICATION

(11) **EP 4 021 588 B1**
(45) Date of publication and mention of the grant of the patent: **04.02.2026**
(21) Application number: 20839550.9
(22) Date of filing: 11.12.2020
(51) Int. Cl.: A61Q 19/10, A61K 8/02, A61K 8/36, A61K 8/41

(54) **PERSONAL CARE COMPOSITIONS**
KÖRPERPFLEGEMITTEL
COMPOSITIONS DE SOINS PERSONNELS

(30) Priority: 30.12.2019 IN 201911054521
(43) Date of publication of application: 06.07.2022
(73) Proprietor: Colgate-Palmolive Company, New York, NY 10022 (US)
(72) Inventor: MALI, Nikhil, Kalyan (W) Maharashtra Mumbai 421 301 (IN); JADHAV, Purushottam, Mumbai 400706 (IN)
(74) Representative: Wichmann, Hendrik
(86) International application number: PCT/US2020/070900
(87) International publication number: WO 2021/138628

(56) References cited:
- EP-A1- 1 798 280
- US-A1- 2004 022 818
- US-A1- 2017 321 169

## Description

### BACKGROUND

Powder soaps normally are produced by preparing an aqueous soap solution, drying the solution by a spray drying method or the like, then adding binder such as water and a non-ionic surfactant, and grinding this into powder with a high-speed mixer. To increase the solubility, the water content of powder soaps prepared in this manner normally is kept to about 7 to 15%. However, such powder soaps have poor flowability and/or feel. Therefore, such powder soaps are preferable only in cases where they are used as clothing detergent that is required high solubility and may be taken out with a measuring cup. To improve flowability, water content is between 1% to 3%. Therefore, a need exists for a powder soap that can exhibit good flowability and good feel while maintaining solubility, thereby making the powder soap suitable for personal care applications. US 2004/0022818 A1 discloses materials and methods for making skin care compositions that contain dried or fresh fruit particles, and also can contain suspending agents, surfactants, emollients, emulsifiers, and/or cationic polymers. EP 1 798 280 A1 discloses a powder soap composition that contains a powder soap made of a fatty acid alkali metal salt. US 2017/321169 A1 discloses a cleaning composition, preferably a granular laundry detergent composition, which contains 1.5 wt % to 10 wt % of soap particles characterized by a particle size distribution with from 35 wt % to 100 wt % of soap particles having particle sizes ranging from about 125 microns to about 355 microns.

### BRIEF SUMMARY

According to some embodiments, the present invention is directed to a powdered soap comprising a plurality of particles, each of the particles comprising: an ionic polymer; a fatty component comprising compounds having C₈ to C₁₈ chains wherein the plurality of particles has an average particle size of at least 600 microns, and wherein the fatty component is ionic as defined in the claims.

Further areas of applicability of the present invention will become apparent from the detailed description provided hereinafter. It should be understood that the detailed description and specific examples, while indicating the preferred embodiment of the invention, are intended for purposes of illustration only and are not intended to limit the scope of the invention, which is defined as set out in the claims.

### DETAILED DESCRIPTION

The following description of the preferred embodiment(s) is merely exemplary in nature and is in no way intended to limit the invention as claimed, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Unless otherwise specified, all percentages and amounts expressed herein and elsewhere in the specification should be understood to refer to percentages by weight. The amounts given are based on the active weight of the material. According to the present application, the term "about" means +/- 5% of the reference value. According to the present application, the term "substantially free" less than about 0.1 wt. % based on the total of the referenced value.

The following description of the preferred embodiment(s) is merely exemplary in nature and is in no way intended to limit the invention, its application, or uses.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

The present invention is directed to a powdered soap as defined in the claims. The soap may be a hand soap and/or body soap and/or hair soap. The soap is a powder soap as defined in the claims - also referred to as a "granular soap." The powder soap is comprised of a plurality of discrete particles having an average particle size as defined in the claims and has a shape.

The shape of each of the plurality of discrete particles may be spherical. In other embodiments, the discrete particles may be ellipsoid, conical, cylindrical, cubical, cuboid, and the like. In some embodiments, the discrete particles may have a non-geometric shape.

The plurality of discrete particles has an average particle size of at least 600 microns as defined in the claims and may have a particle size distribution. The size distribution may include about 8% to about 10% of the plurality of particles having a particle size that is greater than 1,400 microns. The size distribution may include about 76% to about 78% of the plurality of particles having a particle size that is greater than about 840 microns and less than about 1,400 microns. The size distribution may include about 9% to about 10% of the plurality of particles having a particle size that is greater than about 500 microns and less than about 840 microns. The size distribution may include about 0.4% to about 9% of the plurality of particles having a particle size that is greater than about 290 microns and less than about 500 microns. The size distribution may include about 0.6% to about 0.9% of the plurality of particles having a particle size that is less than about 290 microns.

Each particle as defined in the claims comprises a fatty component and may comprise a soap composition comprising a cationic polymer and a clay,. In some embodiments, the soap composition may further comprise a pigment. In some embodiments, the soap composition may further comprise a fragrance.

The cationic polymer may be present in an amount ranging from about 0.009 wt. % to about 4.5 wt. % - including all percentages and sub-ranges-there-between - based on the total weight of the soap composition. Each particle may be formed entirely of the soap composition - therefore, the weight percentages referred to here may also be in reference to the total weight of the respective particle. In a preferred embodiment, the cationic polymer may be present in an amount ranging from about 0.009 wt. % to about 0.9 wt. % - including all percentages and sub-ranges-there-between - based on the total weight of the soap composition.

The cationic polymer may comprise a polyquaternary ammonium - as referred to herein as a "polyquat." The polyquat of the present invention may comprise a compound having formula (I) - which may also be referred to as a "diquat":

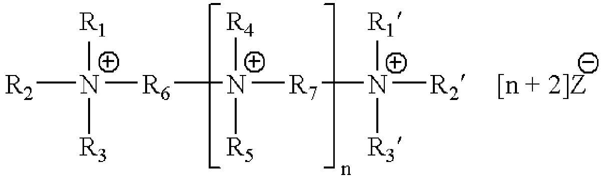

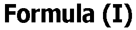

wherein R₁, R₂, R₃, R_{1'}, R_{2'}, R_{3'}, R₄, R₅, in the above formula may be identical or different, and are independently selected from hydrogen, an C₁-C₂₀ alkyl group, an aryl group, a benzyl group, an aralkyl group, or an alkylaryl group. Each C₁-C₂₀alkyl group may be substituted or un-substituted, linear or branched. R₆, and R₇ in the above formula may be identical or different, and are independently selected from (CH₂)ₘ, or (CH₂)ₘ-(CH=CH)ₘ-(CH₂)ₘ, wherein 12=>m>=1, 10=>m'>=1 and 150>=n=>5; and Z is anionic moiety including without limitation, F, Cl, Br, I and COOH.

The polyquat of the present invention may have a weight average molecular weight M_{w} most preferably about 4600 to 11,000. Non-limiting polyquats of the present invention include each of polyquaternium 1-47 compounds.

In a non-limiting example, the polyquat of the present invention may comprise polyquaternium-1: α-4-[1-tris(2-hydroxyethyl) ammonium-2-butenyl] poly[1-dimethylammonium-2-butenyl]-ω-tris(2-hydroxyethyl)ammonium chloride, having the chemical structure described in formula (II):

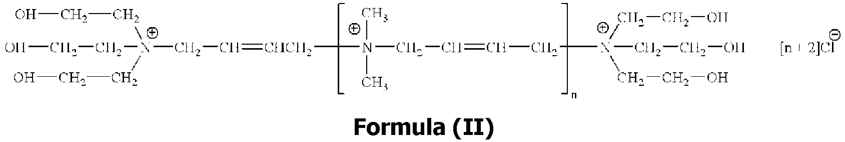

Other non-limiting examples of such polyquaterniums include, but are not limited to (1) the polymeric quaternary ammonium salt of hydroxyethyl cellulose reacted with a trimethyl ammonium substituted epoxide, referred to as Polyquaternium-10; (2) the quaternary ammonium derivative of hydroxypropyl guar, referred to as guar hydroxypropyltrimonium chloride; (3) the copolymer of hydroxyethylcellulose and DADMAC, referred to as Polyquaternium-4; (4) the copolymer of acrylamide and METAMS, referred to as Polyquaternium-5; (5) the homopolymer of DADMAC, referred to as Polyquaternium-6; (6) The copolymer of acrylamide and DADMAC, referred to as Polyquaternium-7; (7) the copolymer of vinyl pyrrolidone and METAMS, referred to as Polyquaternium-11; (8) the homopolymer of METAMS, referred to as Polyquaternium-14; (9) the copolymer of methacrylamide and METAMS, referred to as Polyquaternium-15; (10) the polymeric quatemary ammonium salt of hydroxyethyl cellulose reacted with a lauryl dimethyl ammonium substituted epoxide, referred to as Polyquaternium-24; (11) the copolymer of vinyl pyrrolidone and MAPTAC, referred to as Polyquaternium-28; (12) the copolymer of acrylamide and METAC, referred to as Polyquaternium-32; (13) the copolymer of acrylamide and AETAC, referred to as Polyquaternium-33; (14) the copolymer of butyl methacrylate, dimethylaminoethylmethacrylate, and METAMS, referred to as Polyquaternium-36; (15) the homopolymer of METAC, referred to as Polyquaternium-37; (16) the copolymer of METAMS, methyl methacrylate, and hydroxyethylmethacrylate, referred to as Polyquaternium-45; (17) the homopolymer of MAPTAC, referred to as polymethacrylamidopropyltrimonium chloride; (18) Hydroxypropyl trimethyl ammonium chloride ether derivatives of starch, as generally described by the CAS Registry Number 5670-58-6, the starch of which can be derived from a variety of natural sources such as corn, potato, rice, tapioca, wheat, or other sources; (19) the copolymer of DADMAC and acrylic acid, referred to as Polyquaternium-22; (20) the copolymer of DADMAC, acrylic acid, and acrylamide, referred to as Polyquaternium-39; and (21) the copolymer of MAPTAC, acrylic acid, and methyl(meth)acrylate, referred to at Polyquaternium-47.

In a preferred embodiment, the cationic polymer is the homopolymer of DADMAC, referred to as Polyquaternium-6.

The clay of the present invention may be present in an amount ranging from about 0.05 wt. % to about 2.0 wt. % - including all percentages and sub-ranges-there-between - based on the total weight of the soap composition. Each particle may be formed entirely of the soap composition - therefore, the weight percentages referred to here may also be in reference to the total weight of the respective particle. In a preferred embodiment, the clay may be present in an amount ranging from about 0.1 wt. % to about 1.0 wt. % - including all percentages and sub-ranges-there-between - based on the total weight of the soap composition.

The clay may comprise a one or more of kaolin, kaolinite, dickite, halloysite, nacrite, smectite, montmorillonite, nontronite, illite, bentonite, attapulgite, palygorskite, sepiolite, hormite, pyrophyllite, chlorite, aluminosilicates, and mixtures thereof. In a preferred embodiment, the clay comprises calcined kaolin.

The fatty component of the present invention may be present in an amount ranging from about 94 wt. % to about 98 wt. % - including all percentages and sub-ranges-there-between - based on the total weight of the soap composition. Each particle may be formed entirely of the soap composition - therefore, the weight percentages referred to here may also be in reference to the total weight of the respective particle. In a preferred embodiment, the fatty component may be present in an amount ranging from about 96 wt. % to about 98 wt. % - including all percentages and sub-ranges-there-between - based on the total weight of the soap composition.

The fatty component is ionic as defined in the claims and may be an ionic compound that is a salt of a fatty acid. Non-limiting fatty acids include saturated and unsaturated C₈ to C₃₀ fatty acids. Exemplary fatty acids include, but are not limited to, caprylic acid, capric acid, lauric acid, myristic acid, palmitic acid, palmitoleic acid, stearic acid, oleic acid, ricinoleic acid, vaccenic acid, linoleic acid, α-linolenic acid, γ-linolenic acid, arachidic acid, gadoleic acid, arachidonic acid, behenic acid, erucic acid, lignoceric acid, and mixtures thereof.

In a non-limiting embodiment, the ionic fatty component may comprise a solubilizing cation such as sodium, potassium, ammonium or substituted ammonium.

The fatty component may be present in an amount ranging from about 85 wt. % to about 95 wt. % - based on the total weight of the soap composition - including all percentages and sub-ranges there-between. Each particle may be formed entirely of the soap composition - therefore, the weight percentages referred to here may also be in reference to the total weight of the respective particle. In some embodiments, the fatty component may be present in an amount ranging from about 94 wt. % to about 98 wt. % - based on the total weight of the soap composition - including all percentages and sub-ranges there-between.

According to the present invention, the fatty component may comprise compounds that are a salt of having a C₈ fatty acid - such as caprylic acid. The salts of the C₈ fatty acid may be present in an amount ranging from about 0 wt. % to about 4.5 wt. % based on the total weight of the fatty component - including all amounts and sub-ranges there-between. In some embodiments, the salts of the C₈ fatty acid may be present in an amount ranging from about 0.6 wt. % to about 1 wt. % based on the total weight of the fatty component - including all amounts and sub-ranges there-between. In some embodiments, the salts of the C₈ fatty acid may be present in an amount ranging from about 0.6 wt. % to about 4.5 wt. % based on the total weight of the fatty component - including all amounts and sub-ranges there-between. In some embodiments, the salts of the C₈ fatty acid may be present in an amount of about 0.8 wt. %, based on the total weight of the fatty component. In some embodiments, the salts of the C₈ fatty acid may be present in an amount of about 2.25 wt. %, based on the total weight of the fatty component.

According to the present invention, the fatty component may comprise compounds that are a salt of having a C₁₀ fatty acid - such as capric acid. The salts of the C₁₀ fatty acid may be present in an amount ranging from about 0 wt. % to about 3.6 wt. % based on the total weight of the fatty component - including all amounts and sub-ranges there-between. In some embodiments, the salts of the C₁₀ fatty acid may be present in an amount ranging from about 0.6 wt. % to about 0.9 wt. % based on the total weight of the fatty component - including all amounts and sub-ranges there-between. In some embodiments, the salts of the C₁₀ fatty acid may be present in an amount ranging from about 0.6 wt. % to about 3.6 wt. % based on the total weight of the fatty component - including all amounts and sub-ranges there-between. In some embodiments, the salts of the C₁₀ fatty acid may be present in an amount ranging from about 0.6 wt. % to about 3.6 wt. % based on the total weight of the fatty component - including all amounts and sub-ranges there-between. In some embodiments, the salts of the C₁₀ fatty acid may be present in an amount of about 0.75 wt. %, based on the total weight of the fatty component - including all amounts and sub-ranges there-between. In some embodiments, the salts of the C₁₀ fatty acid may be present in an amount of about 1.8 wt. %, based on the total weight of the fatty component - including all amounts and sub-ranges there-between.

According to the present invention, the fatty component may comprise compounds that are a salt of having a C₁₂ fatty acid - such as lauric acid. The salts of the C₁₂ fatty acid may be present in an amount ranging from about 8.0 wt. % to about 49.5 wt. % based on the total weight of the fatty component - including all amounts and sub-ranges there-between. In some embodiments, the salts of the C₁₂ fatty acid may be present in an amount ranging from about 8.0 wt. % to about 9.2 wt. % based on the total weight of the fatty component - including all amounts and sub-ranges there-between. In some embodiments, the salts of the C₁₂ fatty acid may be present in an amount ranging from about 40.5 wt. % to about 49.5 wt. % based on the total weight of the fatty component - including all amounts and sub-ranges there-between. In other embodiments, the salts of the C₁₂ fatty acid may be present in an amount of about 8.6 wt. %, based on the total weight of the fatty component - including all amounts and sub-ranges there-between. In other embodiments, the salts of the C₁₂ fatty acid may be present in an amount of about 45 wt. %, based on the total weight of the fatty component - including all amounts and sub-ranges there-between.

According to the present invention, the fatty component may comprise compounds that are a salt of having a C₁₄ fatty acid - such as myristic acid. The salts of the C₁₄ fatty acid may be present in an amount ranging from about 2.7 wt. % to about 16.4 wt. % based on the total weight of the fatty component - including all amounts and sub-ranges there-between. In some embodiments, the salts of the C₁₄ fatty acid may be present in an amount ranging from about 2.7 wt. % to about 5.3 wt. % based on the total weight of the fatty component - including all amounts and sub-ranges there-between. In some embodiments, the salts of the C₁₄ fatty acid may be present in an amount ranging from about 11.8 wt. % to about 16.4 wt. % based on the total weight of the fatty component - including all amounts and sub-ranges there-between. In some embodiments, the salts of the C₁₄ fatty acid may be present in an amount of about 4 wt. %, based on the total weight of the fatty component - including all amounts and sub-ranges there-between. In some embodiments, the salts of the C₁₄ fatty acid may be present in an amount of about 14 wt. %, based on the total weight of the fatty component - including all amounts and sub-ranges there-between.

According to the present invention, the fatty component may comprise compounds that are a salt of having a C₁₆ fatty acid - such as palmitic acid. In some embodiments, the salts of the C₁₆ fatty acid may be present in an amount ranging from about 10 wt. % to about 44.1 wt. % based on the total weight of the fatty component - including all amounts and sub-ranges there-between. In some embodiments, the salts of the C₁₆ fatty acid may be present in an amount ranging from about 34.9 wt. % to about 44.1 wt. % based on the total weight of the fatty component - including all amounts and sub-ranges there-between. In some embodiments, the salts of the C₁₆ fatty acid may be present in an amount ranging from about 10 wt. % to about 14.6 wt. % based on the total weight of the fatty component - including all amounts and sub-ranges there-between. In some embodiments, the salts of the C₁₆ fatty acid may be present in an amount of about 39.5 wt. %, based on the total weight of the fatty component. In some embodiments, the salts of the C₁₆ fatty acid may be present in an amount of about 12.2 wt. %, based on the total weight of the fatty component.

According to the present invention, the fatty component may comprise compounds that are a salt of having a C₁₈ fatty acid - such as stearic acid, oleic acid, linoleic acid, linolenic acid, and mixtures thereof. In some embodiments, the salts of the C₁₈ fatty acid may be present in an amount ranging from about 15 wt. % to about 52 wt. % based on the total weight of the fatty component - including all amounts and sub-ranges there-between.

The C₁₈ fatty acid may be entirely saturated - such as stearic acid - whereby the salt of stearic acid is present in an amount ranging from about 1.7 wt. % to about 14.1 wt. % based on the total weight of the fatty component - including all amounts and sub-ranges there-between. In some embodiments, the salt of stearic acid is present in an amount ranging from about 1.7 wt. % to about 4.2 wt. % based on the total weight of the fatty component - including all amounts and sub-ranges there-between. In some embodiments, the salt of stearic acid is present in an amount ranging from about 6.7 wt. % to about 14.1 wt. % based on the total weight of the fatty component - including all amounts and sub-ranges there-between. In some embodiments, the salt of stearic acid is present in an amount of about 2.96 wt. %, based on the total weight of the fatty component. In some embodiments, the salt of stearic acid is present in an amount of about 10.4 wt. %, based on the total weight of the fatty component.

The C₁₈ fatty acid may be partially unsaturated having a single unsaturated C=C bond - such as oleic acid - whereby the salt of oleic acid is present in an amount ranging from about 12 wt. % to about 32.1 wt. % based on the total weight of the fatty component - including all amounts and sub-ranges there-between. In some embodiments, the salt of oleic acid is present in an amount ranging from about 21.5 wt. % to about 32.1 wt. % based on the total weight of the fatty component - including all amounts and sub-ranges there-between. In some embodiments, the salt of oleic acid is present in an amount ranging from about 12 wt. % to about 21.75 wt. % based on the total weight of the fatty component - including all amounts and sub-ranges there-between. In some embodiments, the salt of oleic acid is present in an amount of about 26.8 wt. %, based on the total weight of the fatty component. In some embodiments, the salt of oleic acid is present in an amount of about 16.9 wt. %, based on the total weight of the fatty component.

The C₁₈ fatty acid may be partially unsaturated having a two unsaturated C=C bonds - such as linoleic acid - whereby the salt of linoleic acid is present in an amount ranging from about 1.42 wt. % to about 5.8 wt. % based on the total weight of the fatty component - including all amounts and sub-ranges there-between. In some embodiments, the salt of linoleic acid is present in an amount ranging from about 2.2 wt. % to about 5.8 wt. % based on the total weight of the fatty component - including all amounts and sub-ranges there-between. In some embodiments, the salt of linoleic acid is present in an amount ranging from about 1.4 wt. % to about 3.7 wt. % based on the total weight of the fatty component - including all amounts and sub-ranges there-between. In some embodiments, the salt of linoleic acid is present in an amount of about 2.53 wt. %, based on the total weight of the fatty component. In some embodiments, the salt of linoleic acid is present in an amount of about 4 wt. %, based on the total weight of the fatty component.

The C₁₈ fatty acid may be partially unsaturated having a three unsaturated C=C bonds - such as linolenic acid - whereby the salt of linolenic acid is present in an amount ranging from about 0 wt. % to about 0.92 wt. % based on the total weight of the fatty component - including all amounts and sub-ranges there-between. In some embodiments, the salt of linolenic acid is present in an amount ranging from about 0 wt. % to about 0.25 wt. % based on the total weight of the fatty component - including all amounts and sub-ranges there-between. In some embodiments, the salt of linolenic acid is present in an amount of about 0.125 wt. %, based on the total weight of the fatty component. In some embodiments, the salt of linolenic acid is present in an amount of about 0.46 wt. %, based on the total weight of the fatty component.

In a non-limiting example, the fatty component may comprise salts of the following fatty acids in the respective amounts based on the total weight of the fatty component: 0.8 wt. % of a salt of caprylic acid; 0.75 wt. % of a salt of capric acid; 8.6 wt. % of a salt of lauric acid; 4.0 wt. % of a salt of myristic acid; 39.5 wt. % of a salt of palmitic acid; 10.4 wt. % of a salt of stearic acid; 26.8 wt. % of a salt of oleic acid; 4.0 wt. % of a salt of linoleic acid; and 0.125 wt. % of a salt of linolenic acid.

In a non-limiting example, the fatty component may comprise salts of the following fatty acids in the respective amounts based on the total weight of the fatty component: 0.8 wt.% or 2.25 wt. % of a salt of caprylic acid; 0.75 wt.% or 1.8 wt. % of a salt of capric acid; 8.6 wt. % or 45 wt.% of a salt of lauric acid; 4 wt. % or 14 wt. % of a salt of myristic acid; 12.2 wt. % or 39.5 wt. % of a salt of palmitic acid; 2.96 wt. % or 10.4 wt. % of a salt of stearic acid; 16.9 wt. % or 26.8 wt. % of a salt of oleic acid; 2.53 wt. % or 4 wt. % of a salt of linoleic acid; and 0.125 wt. % or 0.46 wt. % of a salt of linolenic acid.

The soap composition may further comprise one or more fragrances. The fragrance may be present in an amount ranging from about 1 wt. % to about 1.5 wt. % - based on the total weight of the soap composition - including all percentages and sub-ranges there-between. Each particle may be formed entirely of the soap composition - therefore, the weight percentages referred to here may also be in reference to the total weight of the respective particle. In some embodiments, the fragrance may be present in an amount ranging from about 0.5 wt. % to about 1.7 wt. % - based on the total weight of the soap composition - including all percentages and sub-ranges there-between.

Non-limiting examples of fragrances and perfumes include odor compounds selected from: 7-acetyl-1,2,3,4,5,6,7,8-octahydro-1,1,6,7-tetramethylnaphthalene, α-ionone, β-ionone, γ-ionone α-isomethylionone, methylcedrylone, methyl dihydrojasmonate, methyl 1,6,10-trimethyl-2,5,9-cyclododecatrien-1-yl ketone, 7-acetyl-1,1,3,4,4,6-hexamethyltetralin, 4-acetyl-6-tert-butyl-1,1-dimethylindane, hydroxyphenyl butanone, benzophenone, methyl β-naphthyl ketone, 6-acetyl-1,1,2,3,3,5-hexamethylindane, 5-acetyl-3-isopropyl-1,1,2,6-tetramethylindane, 1-dodecanal, 4-(4-hydroxy-4-methylpentyl)-3-cyclohexene-1-carboxaldehyde, 7-hydroxy-3,7-dimethyloctanal, 10-undecen-1-al, isohexenylcyclohexylcarboxaldehyde, formyltricyclodecane, condensation products of hydroxycitronellal and methyl anthranilate, condensation products of hydroxycitronellal and indole, condensation products of phenylacetaldehyde and indole, 2-methyl-3-(para-tert-butylphenyl)propionaldehyde, ethylvanillin, heliotropin, hexylcinnamaldehyde, amylcinnamaldehyde, 2-methyl-2-(isopropylphenyl)propionaldehyde, coumarin, γ-decalactone, cyclopentadecanolide, 16-hydroxy-9-hexadecenoic acid lactone, 1,3,4,6,7,8-hexahydro-4,6,6,7,8,8-hexamethylcyclopenta-γ-2-benzopyran, β-naphthol methyl ether, ambroxane, dodecahydro-3*a*,6,6,9*a*-tetramethylnaphtho[2,1*b*]furan, cedrol, 5-(2,2,3-trimethylcyclopent-3-enyl)-3-methylpentan-2-ol, 2-ethyl-4-(2,2,3-trimethyl-3-cyclopenten-1-yl)-2-buten-1-ol, caryophyllene alcohol, tricyclodecenyl propionate, tricyclodecenyl acetate, benzyl salicylate, cedryl acetate, and tert-butylcyclohexyl acetate.

Other fragrances may include odor compounds selected from essential oils, resinoids and resins from a large number of sources, such as, for example, Peru balsam, olibanum resinoid, styrax, labdanum resin, nutmeg, cassia oil, benzoin resin, coriander, and lavandin.

Further suitable fragrances include odor compounds selected from phenylethyl alcohol, terpineol, linalool, linalyl acetate, geraniol, nerol, 2-(1,1-dimethylethyl)cyclo-hexanol acetate, benzyl acetate, and eugenol. The fragrances or perfumes can be used as single substances or in a mixture with one another.

The soap composition may further comprise one or more colorants. The colorants may be a pigment, a dye, or mixtures thereof. Non-limiting examples of pigments include titanium dioxide, Zinc Oxide, Kaolin, Mica etc. Non-limiting examples of dyes include food dyes suitable for food, drug and cosmetic applications, and mixtures thereof. Some color agents (colorants) are known as FD&C dyes.

The colorants may be present in an amount ranging from about 0.0001 wt. % to about 0.4 wt. % - based on the total weight of the soap composition - including all percentages and sub-ranges there-between. Each particle may be formed entirely of the soap composition - therefore, the weight percentages referred to here may also be in reference to the total weight of the respective particle. In some embodiments, the colorants may be present in an amount ranging from about 0.0001 wt. % to about 4 wt. % - based on the total weight of the soap composition - including all percentages and sub-ranges there-between.

The discrete particles that form the powder soap of the present invention may have a content of liquid carrier - such as water or organic solvents - that is less than about 9 wt. % based on the total weight of each discrete particle. In some embodiments, the discrete particles that form the powder soap of the present invention may have a content of liquid carrier - such as water or organic solvents - that is less than about 7 wt. % based on the total weight of each discrete particle. In some embodiments, the discrete particles that form the powder soap of the present invention may have a content of liquid carrier - such as water or organic solvents - that is less than about 5 wt. % based on the total weight of each discrete particle.

The discrete particles that form the powder soap of the present invention may be provided as solid discrete particles. In particular, the discrete particles may have a solids content of at least about 90% based on the total weight of each discrete particle. Preferably, the discrete particles may have a solids content of at least about 95% based on the total weight of each discrete particle. Preferably, the discrete particles may have a solids content of at least about 96% based on the total weight of each discrete particle.

The soap composition present in the powder soap of the present invention may have a solids content of at least about 97% based on the total weight of the soap composition. Preferably, the soap composition present in the powder soap may have a solids content of at least about 98% based on the total weight of the soap composition. Preferably, the soap composition present in the powder soap may have a solids content of at least about 99% based on the total weight of the soap composition.

The soap composition present in the powder soap may have a content of liquid carrier - such as water or organic solvents - that is less than about 3 wt. % based on the total weight of the soap composition. In some embodiments, the soap composition present in the powder soap of the present invention may have a content of liquid carrier - such as water or organic solvents - that is less than about 2 wt. % based on the total weight of the soap composition. In some embodiments, the soap composition present in the powder soap may have a content of liquid carrier - such as water or organic solvents - that is less than about 1 wt. % based on the total weight of each soap composition.

In some embodiments, the soap composition that forms the powder soap of the present invention may be substantially free of liquid carrier - such as water or organic solvents.

The powder soap of the present invention may be manufactured by forming a blend of the cationic polymer, the clay, and the fatty component. According to the embodiments containing colorant, fragrances, and other additives, the additional components may also be added to the blend. The blend may be lightly agitated until the various components are uniformly distributed throughout the blend. According to some embodiments, the blend may further comprise one or more liquid carriers (e.g., water, non-aqueous solvent) to help the agitation of the various components.

Once agitated, the blend may be dried to remove the liquid carrier. The resulting dry blend may then be subjected to mechanical stress (e.g., grinding), thereby breaking apart the blend into smaller particles that form the plurality of discrete particles of the powder soap. Non-limiting examples of grinding may be subjecting the dry blend to a high-speed mixer, a Henschel mixer, or a Loedige mixer.

After grinding, the plurality of discrete particles may be collected. In some embodiments, the plurality of particles may be subjected to a screening to remove small or large particles not suitable for the powder soap. In other embodiments, the plurality of particles are not subjected to a screening process.

The resulting powder soap may then be distributed into the reservoir of a soap dispensing device. In non-limiting examples, the soap dispensing device may be a hermetically sealable bag, box, or other container.

## Claims

1. A powdered soap comprising a plurality of particles, each of the particles comprising:
an ionic polymer; and
a fatty component comprising compounds having C₈ to C₁₈ chains;
wherein the plurality of particles has an average particle size of at least 600 microns, and
wherein the fatty component is ionic.

2. The powdered soap according to claim 1, wherein the fatty component comprises compounds having C₁₈ chains in an amount ranging from 15 wt. % to 52 wt. % based on the total weight of the fatty component.

3. The powdered soap according to any foregoing claim, wherein the fatty component comprises compounds having C₁₆ chains in an amount ranging from 10 wt. % to 44.1 wt. % based on the total weight of the fatty component.

4. The powdered soap according to any foregoing claim, wherein the fatty component comprises compounds having C₁₄ chains in an amount ranging from 2.7 wt. % to 16.4 wt. % based on the total weight of the fatty component.

5. The powdered soap according to any foregoing claim, wherein the fatty component comprises compounds having C₁₂ chains in an amount ranging from 8 wt. % to 49.5 wt. % based on the total weight of the fatty component.

6. The powdered soap according to any foregoing claim, wherein the fatty component comprises compounds having C₁₀ chains in an amount up to 3.6 wt. % based on the total weight of the fatty component.

7. The powdered soap according to any foregoing claim, wherein the fatty component comprises compounds having C₈ chains in an amount up to 4.5 wt. % based on the total weight of the fatty component.

8. The powdered soap according to any foregoing claim, wherein the particles further comprise clay.

9. The powdered soap according to any foregoing claim, wherein the fatty component is present in an amount ranging from 85 wt. % to 95 wt. % based on the total weight of each of the particle.

10. A personal care composition comprising:
a plurality of particles, wherein each particle comprises:
an ionic polymer; and
a fatty component comprising compounds having C₈ to C₁₈ chains;
wherein the plurality of particles has an average particle size of at least 600 microns, and
wherein the fatty component is ionic.

11. The personal care composition according to claim 10, further comprising a fragrance.

12. The personal care composition according to any one of claims 10 or 11, wherein each particle further comprises titanium dioxide or any other suitable pigment, and optionally wherein the titanium dioxide is present in an amount ranging from 0.1 wt. % to 0.5 wt. % based on the total weight of each particle.

13. The personal care composition according to any one of claims 10 to 12, wherein the personal care composition is in a form selected from: a hand soap; a body cleanser; a shampoo; a conditioner; and a scrub.

14. A method of cleansing a keratinous surface comprising applying a soap according to any one of claims 1 to 9, or a personal care composition according to any one of claims 10 to 13, to a keratinous surface of a subject in need thereof; and optionally rinsing said keratinous surface.

15. The method according to claim 14, wherein the method does not include a rinsing step; and optionally wherein the keratinous surface is selected from: skin; hair; a nail; and a combination of two or more thereof.

## Patentansprüche

1. Seifenpulver, das eine Vielzahl von Partikeln umfasst, wobei jedes der Partikel Folgendes umfasst:
ein ionisches Polymer; und
eine Fettkomponente, die Verbindungen mit C₈- bis C₁₈-Ketten umfasst;
wobei die Vielzahl von Partikeln eine durchschnittliche Partikelgröße von mindestens 600 Mikrometern aufweist und
wobei die Fettkomponente ionisch ist.

2. Seifenpulver nach Anspruch 1, wobei die Fettkomponente Verbindungen mit C₁₈-Ketten in einer Menge von 15 Gew.-% bis 52 Gew.-%, bezogen auf das Gesamtgewicht der Fettkomponente, umfasst.

3. Seifenpulver nach einem der vorstehenden Ansprüche, wobei die Fettkomponente Verbindungen mit C₁₆-Ketten in einer Menge von 10 Gew.-% bis 44,1 Gew.-%, bezogen auf das Gesamtgewicht der Fettkomponente, umfasst.

4. Seifenpulver nach einem der vorstehenden Ansprüche, wobei die Fettkomponente Verbindungen mit C₁₄-Ketten in einer Menge von 2,7 Gew.-% bis 16,4 Gew.-%, bezogen auf das Gesamtgewicht der Fettkomponente, umfasst.

5. Seifenpulver nach einem der vorstehenden Ansprüche, wobei die Fettkomponente Verbindungen mit C₁₂-Ketten in einer Menge von 8 Gew.-% bis 49,5 Gew.-%, bezogen auf das Gesamtgewicht der Fettkomponente, umfasst.

6. Seifenpulver nach einem der vorstehenden Ansprüche, wobei die Fettkomponente Verbindungen mit C₁₀-Ketten in einer Menge von bis zu 3,6 Gew.-%, bezogen auf das Gesamtgewicht der Fettkomponente, umfasst.

7. Seifenpulver nach einem der vorstehenden Ansprüche, wobei die Fettkomponente Verbindungen mit C₈-Ketten in einer Menge von bis zu 4,5 Gew.-%, bezogen auf das Gesamtgewicht der Fettkomponente, umfasst.

8. Seifenpulver nach einem der vorstehenden Ansprüche, wobei die Partikel zusätzlich Ton enthalten.

9. Seifenpulver gemäß einem der vorstehenden Ansprüche, wobei die Fettkomponente in einer Menge von 85 Gew.-% bis 95 Gew.-%, bezogen auf das Gesamtgewicht jedes der Partikel, vorhanden ist.

10. Körperpflegezusammensetzung, umfassend:
eine Vielzahl von Partikeln, wobei jedes Partikel Folgendes umfasst:
ein ionisches Polymer; und
eine Fettkomponente, die Verbindungen mit C₈- bis C₁₈-Ketten umfasst;
wobei die Vielzahl von Partikeln eine durchschnittliche Partikelgröße von mindestens 600 Mikrometern aufweist und
wobei die Fettkomponente ionisch ist.

11. Körperpflegezusammensetzung nach Anspruch 10, die ferner einen Duftstoff umfasst.

12. Körperpflegezusammensetzung gemäß einem der Ansprüche 10 oder 11, wobei jedes Partikel ferner Titandioxid oder ein anderes geeignetes Pigment umfasst und wobei das Titandioxid optional in einer Menge von 0,1 Gew.-% bis 0,5 Gew.-%, bezogen auf das Gesamtgewicht jedes der Partikel, vorhanden ist.

13. Körperpflegezusammensetzung gemäß einem der Ansprüche 10 bis 12, wobei die Körperpflegezusammensetzung in einer Form vorliegt, die ausgewählt ist aus: einer Handseife; einem Körperreinigungsmittel; einem Shampoo; einer Haarspülung; und einem Peeling.

14. Verfahren zum Reinigen einer keratinhaltigen Oberfläche, umfassend das Auftragen einer Seife gemäß einem der Ansprüche 1 bis 9 oder einer Körperpflegezusammensetzung gemäß einem der Ansprüche 10 bis 13 auf eine keratinhaltige Oberfläche eines Subjekts, das dies benötigt; und optional das Abspülen der keratinhaltigen Oberfläche.

15. Verfahren nach Anspruch 14, wobei das Verfahren keinen Spülschritt umfasst und wobei die keratinhaltige Oberfläche optional ausgewählt ist aus: Haut, Haar, Nagel und einer Kombination aus zwei oder mehr davon.

## Revendications

1. Savon en poudre comprenant une pluralité de particules, chacune des particules comprenant :
un polymère ionique ; et
un constituant gras comprenant des composés ayant des chaînes en C₈ à C₁₈ ;
dans lequel la pluralité de particules a une granulométrie moyenne d'au moins 600 microns, et
dans lequel le constituant gras est ionique.

2. Savon en poudre selon la revendication 1, dans lequel le constituant gras comprend des composés ayant des chaînes en C₁₈ en une quantité comprise entre 15 % en poids et 52 % en poids par rapport au poids total du constituant gras.

3. Savon en poudre selon l'une quelconque des revendications précédentes, dans lequel le constituant gras comprend des composés ayant des chaînes en C₁₆ en une quantité comprise entre 10 % en poids et 44,1 % en poids par rapport au poids total du constituant gras.

4. Savon en poudre selon l'une quelconque des revendications précédentes, dans lequel le constituant gras comprend des composés ayant des chaînes en C₁₄ en une quantité comprise entre 2,7 % en poids et 16,4 % en poids par rapport au poids total du constituant gras.

5. Savon en poudre selon l'une quelconque des revendications précédentes, dans lequel le constituant gras comprend des composés ayant des chaînes en C₁₂ en une quantité comprise entre 8 % en poids et 49,5 % en poids par rapport au poids total du constituant gras.

6. Savon en poudre selon l'une quelconque des revendications précédentes, dans lequel le constituant gras comprend des composés ayant des chaînes en C₁₀ en une quantité allant jusqu'à 3,6 % en poids par rapport au poids total du constituant gras.

7. Savon en poudre selon l'une quelconque des revendications précédentes, dans lequel le constituant gras comprend des composés ayant des chaînes en C₈ en une quantité allant jusqu'à 4,5 % en poids par rapport au poids total du constituant gras.

8. Savon en poudre selon l'une quelconque des revendications précédentes, dans lequel les particules comprennent en outre de l'argile.

9. Savon en poudre selon l'une quelconque des revendications précédentes, dans lequel le constituant gras est présent en une quantité comprise entre 85 % en poids et 95 % en poids par rapport au poids total de chaque particule.

10. Composition de soins personnels comprenant :
une pluralité de particules, dans laquelle chaque particule comprend :
un polymère ionique ; et
un constituant gras comprenant des composés ayant des chaînes en C₈ à C₁₈ ;
dans laquelle la pluralité de particules a une granulométrie moyenne d'au moins 600 microns, et
dans laquelle le constituant gras est ionique.

11. Composition de soins personnels selon la revendication 10, comprenant en outre un parfum.

12. Composition de soins personnels selon l'une quelconque des revendications 10 ou 11, dans laquelle chaque particule comprend en outre du dioxyde de titane ou tout autre pigment approprié, et éventuellement dans laquelle le dioxyde de titane est présent en une quantité comprise entre 0,1 % en poids et 0,5 % en poids par rapport au poids total de chaque particule.

13. Composition de soins personnels selon l'une quelconque des revendications 10 à 12, dans laquelle la composition de soins personnels se présente sous une forme choisie parmi : un savon pour les mains ; un nettoyant pour le corps ; un shampoing ; un après-shampoing ; et un gommage.

14. Procédé de nettoyage d'une surface kératinique comprenant l'application d'un savon selon l'une quelconque des revendications 1 à 9, ou d'une composition de soins personnels selon l'une quelconque des revendications 10 à 13, sur une surface kératinique d'un sujet en ayant besoin ; et éventuellement le rinçage de ladite surface kératinique.

15. Procédé selon la revendication 14, dans lequel le procédé n'inclut pas d'étape de rinçage ; et éventuellement dans lequel la surface kératinique est choisie parmi : la peau ; les cheveux ; un ongle ; et une combinaison de deux ou plus de ceux-ci.
